Europäisches Patentamt

European Patent Office

Office européen des brevets

(11) Publication number: **0 155 172**
A2

# (12) EUROPEAN PATENT APPLICATION

(21) Application number: **85301679.8**

(22) Date of filing: **11.03.85**

(51) Int. Cl.⁴: **C 12 P 21/00, C 12 N 5/00**

(30) Priority: **12.03.84 JP 46684/84**

(43) Date of publication of application: **18.09.85**
**Bulletin 85/38**

(84) Designated Contracting States: **DE FR GB**

(71) Applicant: **KYOWA HAKKO KOGYO KABUSHIKI KAISHA, 6-1, Ohte-machi 1-chome, Chiyoda-ku Tokyo 100 (JP)**

(72) Inventor: **Yoshida, Hajime, 9-18 Isobe, Sagamihara-shi Kanagawa-ken (JP)**
Inventor: **Hanai, Nobuo, 3-7-10, Yurigaoka Asoa-ku, Kawasaki-shi Kanagawa-ken (JP)**

(74) Representative: **Hildyard, Edward Martin et al, Frank B. Dehn & Co. European Patent Attorneys Imperial House 15-19 Kingsway, London WC2B 6UZ (GB)**

(54) **Anti-human lung cancer monoclonal antibodies.**

(57) A human lung tumour-specific monoclonal antibody which binds to an antigen specific to human lung tumour but not to an antigen of normal human tissue of the lung or to an antigen of other organs, may be made by culturing a suitably selected hybridoma made by fusing antibody-producing cells from an animal immunised with human lung cancer antigens, with myeloma cells.

EP 0 155 172 A2

1FB147-218

## Anti-Human Lung Cancer Monoclonal Antibodies

The present invention relates to monoclonal antibodies. In particular, it relates to monoclonal antibodies capable of binding to human lung tumours, but not normal human tissues (hereinafter referred to as anti-human lung cancer monoclonal antibodies).

It was previously known to immunize an animal with antigens originating from a human lung tumour to obtain an antiserum for human lung tumour cells. Such antiseara are, however, also capable of binding to normal human cells. Following the finding by Köhler and Milsten (Nature 256, 495-497, 1975) that monoclonal antibodies can be obtained by fusing antibody-forming cells derived from an immunized animal with myeloma cells to form hybridoma cells and culturing the resultant hybridoma cells, it has more recently been reported that monoclonal antibodies for human lung tumours may be obtained by such a method [Cancer Res., 42, 3187 (1982); cancer Res., 42, 150 (1982); J. Surgical Res., 30, 403 (1981); Transplantation Proceed., XIII (4), 1942 (1981); J. Immunol., 131 (1) 497 (1983); Meneki Gakkai Yoshi, P. 212 (Title No. 107), (1983), Kyoizumi Seishi in Japanese version]. However, nearly all of the known monoclonal antibodies capable of binding to human lung tumours are also reactive with human tumours other than human lung tumours and many are, in addition, reactive with human normal cells. Among the known monoclonal antibodies capable of binding to human lung tumours, for example, one reported in J. Immunol., 131, (1) 497 (1983) is said to be relatively specific for lung cancer. However, using this monoclonal antibody or such other similar known monoclonal antibodies, although it is possible to distinguish the small cell cancer from other cancers, it is difficult to recognize the tissue-types of the lung cancer, such as squamous cell carcinoma and adenocarcinoma. Moreover, such monoclonal antibodies have in general only been tested with a relatively small number of cell types. Before a monoclonal antibody can be characterised as a lung tumour specific monoclonal antibody, its binding with a wide variety of normal tissues as well as with a wide varieity of tumours other than lung tumours should be studied. In our view, monoclonal antibodies which may be used successfully in practice for diagnosis and treatment of human lung cancers are not yet known in the art.

In one aspect, our invention provides a human lung tumour-specific monoclonal antibody which binds to an antigen specific to human lung tumour but not to an antigen of normal human tissue of the lung or to an antigen of other organs.

Hybridoma cells which secrete a monoclonal antibody according to the present invention, may be prepared by a process comprising immunizing a mammal with human lung cancer antigens originating from human lung squamous cell carcinoma and/or human lung adenocarcinoma, fusing antibody-forming cells obtained from said immunized mammals with non-immunoglobulin-secreting myeloma cells to produce hybridoma cells and selecting from the resultant hybridoma cells those which produce monoclonal antibodies having the desired binding specificity. The selected hybridoma cells may then either be cultured in vitro in a medium, or administered to a syngenic animal intraperitoneally to accumulate ascites therein; finally the monoclonal antibodies are recovered from the cultured broth or ascites.

The monoclonal antibodies of the present invention may be prepared, for example, in the following manner. Although the methods of immunization, hybridoma preparation and selection, and hybridomic culturing described below have been found to give good results, our invention is not restricted to the use of these procedures.

(1)  Immunization and preparation of antibody-forming cells

An animal of age 3-10 weeks (preferably about 8 weeks) is immunized with· a human cancer antigen such as e.g. whole cells, broken cells or membrane fragments obtained from human lung squamous cell carcinoma or from human lung adenocarcinoma to develop in the animal antibody-forming cells which may be isolated as, for example, spleen cells, lymph nodes, or peripheral blood from the immunized animal.  Immunization may be effected subcutaneously, intravenously or abdominally using said antigen e.g. at a dose of $10^6-10^7$ cells per animal.  When membrane fragments or broken cells are used, the dose may e.g. be 10-500 mcg per animal, togther with a suitable adjuvant such as, for example, Freund's complete adjuvant, aluminium hydroxide gel or pertussis adjuvant.  Immunization may be repeated 2-5 times at intervals of 1-2 weeks. Advantageously, every 3-7 days after immunization, blood may be collected from the orbital plexus vein of the animal to assay the reactivity of the resultant antiserum with human lung squamous cell carcinoma antigen and/or human lung adenocarcinoma antigen, using an enzyme linked immunosorbent assay method as follows [Koso Meneki Sokutei-ho (ELISA), published by Igaku Shoin, Tokyo (1976)].

Enzyme-linked immunosorbent assay method:

A membrane fragment preparation obtained from either normal cells or tumour cells (containing typically 10-1000 mcg/ml of protein) is poured into each well of an ELISA plate with 96 wells [Flow Laboratories, U.S.A.) in an amount of 100-200 mcl per well and allowed to stand at 4°C over 1-2 nights.  Then, bovine serum albumin (BSA) is coated on the bottom surface of each well. After removal of supernatant from each well, each well is washed thoroughly with deionized water or a PBS solution [disodium phosphate 1.83g; mono-potassium phosphate 0.21 g; sodium chloride 7.65 g; deionized water 1 l; pH=7.2]. Then, a 1% BSA-PBS solution (100-200 mcl) is poured into each well and the plate is allowed to stand at 4°C over 1-2 nights. This treatment with BSA-PBS solution prevents interference of the assay by non-specific binding. After removal of the BSA-PBS solution, each well is washed thoroughly with deionized water or a PBS solution. A primary antibody [mouse serum, supernatant of the culture of the hybridoma cells or a crude monoclonal antibody] diluted with a BSA-PBS solution is poured into each well in an amount of 100 mcl per well and allowed to stand at 4°C overnight. After washing once with deionized water and 6 times with 2M NaCl solution, a secondary antibody (e.g. antimouse immunoglobulin IgG or F(ab')$_2$ -urease combined product [Commonwealth Serum Limited, Australia, hereinafter referred to as CSL], X100; 100 mcl per well) is added to each well and the plate is allowed to stand at room temperature for 2 hours. After washing 3 times with deionised water, a urease substrate solution (100 mcl per well; Commonwealth Serum Limited) is poured into each well and the plate is allowed to stand at room temperature for 10-30 minutes. Then, the reaction is discontinued by adding 1 % methylthiolate (20 mcl per well).

The antibody titre is assayed by colorimetric determination at 600 nm. An animal which is strongly reactive with a human lung cancer antigen originating from human lung squamous cell carcinoma or human lung adenocarcinoma is deemed to have been efficiently immunized with the human lung cancer antigen and may be used as the source of antibody-producing cells

needed for the preparation of the hybridoma cells of the present invention.

When whole cells are used as antigen for carrying out the enzyme-linked immunosorbent assay, one may for example use a Falcon 3072 plate for culturing the target cells. After addition of a 0.25 % glutaraldehyde-PBS solution, the plate is allowed to stand at room temperature for 1-2 hours. After washing thoroughly with a PBS solution, a 1 % BSA-PBS solution (100-200 mcl per well) is added to the plate which is then allowed to stand for 2 hours. The plate is then washed thoroughly with deionized water or a PBS solution and the antibody titre is determined in conventional manner for antigen-coated plates (cf the method described above).

It is preferred, 3-4 days before isolation of antibody-forming cells, to administer one member selected whole tumour cells, broken cells or membrane fragments from a human lung tumour ($2-5 \times 10^6$ cells or 20-400 mcg per animal) abdominally to the immunized mouse.

The preferred antibody-forming cells are spleen cells. The spleen tissue may be removed and cut into small pieces in a MEM medium (Nissui Seiyaku K.K., Japan). The cells are loosened by using a pincette and centrifuged (1200 r.p.m./5 min.) to remove supernatant. The remaining material is treated with a tris-ammonium chloride buffer solution (pH=7.65) for 1-2 minutes to remove the red cells. After washing with MEM medium 3 times, the resultant spleen cells may be used for fusion.

(2)	Preparation of myeloma cells

Preferred myeloma cell lines for the purpose of the present invention include, for example, myeloma cell lines of mouse origin such as the myeloma cell lines from 8-azaguanine-resistant BALB/c mouse, such

as P3-X63-Ag8-U1 (P3-U1) [Current Topics in Microbiology and Immunology-1] [Euro. J. Immunology, 6, 511-519 (1976)], SP2/0-Ag14 (SP-2) [Nature, 276, 269-270 (1978)], P3-X63-Ag8.653 (653) [J. Immunol. 123, 1548-1550 (1979)], P3-X63-Ag8 (X63) [Nature, 256, 495-497 (1975)] and the like. These cell lines may be subcultured by the use of 8-azaguanine-resistant medium [prepared by adding 8-azaguanine (15 mcg/ml) to RPM1-1640 normal medium further containing glutamine (1.5 mM), 2-mercaptoethanol (5 x $10^{-5}$M), gentamycin (10 mcg/ml) and foetal calf serum (FCS; 10%; commercial product of Commonwealth Serum Limited, Australia, hereinafter referred to as CSL). It is preferred to ensure an adequate population of myeloma cells by subculturing the cells in a normal medium for 3-4 days before the hybridization.

(3)  Cell fusion and selection of hybridoma cells

Both the antibody-forming cells obtained by step (1) and the myeloma cells obtained by step (2) are thoroughly washed with MEM medium or a PBS solution and mixed together at a ratio of 5-10:1. The mixture is centrifuged (1200 r.p.m./5 min.). After removal of supernatant, the precipitated cells are well loosened. To the mixed cells a mixture of polyethylene glycol 1000 (PEG 1000; 2 g), MEM (2 ml) and dimethylsulfoxide (0.7 ml) is added at 37°C with stirring in an amount of 0.2-1 ml per $10^3$ antibody-forming cells. Then, 1-2 ml of MEM medium is added to the mixture several times at intervals of 1-2 minutes, and finally MEM medium is added to the mixture to make up the total volume to 50 ml. The mixture is centrifuged (900 r.p.m./ 5 min.). After removal of supernatant, the cells are loosened. A normal medium (RPMI 1640 medium containing 10% FCS) (100 ml) is added to the loosened cells and they are suspended freely by the use of a Mohr pipette.

The cell suspension is added to each well of a culture plate having 24 wells in an amount of 1 ml per well and cultured at 37°C for 24 hours in an incubator containing 5% $CO_2$. A HAT medium [prepared by adding to a normal medium, hypoxanthine ($10^{-4}$M), thymidine ($1.5 \times 10^{-5}$M) and aminopterin ($4 \times 10^{-7}$M)] is then added in an amount of 1 ml per well and culturing is continued for a further 24 hours. Following this, for 2 days, every 24 hours 1 ml of the supernatant is removed from each culture and replaced by the same amount of new HAT medium. Culturing is continued at 37°C for 10-14 days in a $CO_2$ incubator. The wells having growing colonies of hybridoma cells are selected.

From each of the selected wells, a portion of supernatant (1 ml) is removed and replaced by a same amount of new HT medium [prepared by removing aminopterin from HAT medium]. The process is repeated every 24 hours for 2 days. After culturing for 3-4 days in HT medium, a portion of the supernatant is collected from each culture and the antibody titre of antibodies to human lung squamous cell carcinoma or human lung adenocarcinoma is determined by the above-described enzyme-linked immunosorbent assay. At this time, the reactivities of the supernatants with normal cell antigens is determined by a similar method.

The hybridomas in those wells which are strongly reactive with the antigen present in whole tumour cell, broken cells or membrane fragments of such cells from human lung squamous cell carcinoma or human lung adenocarcinoma and which are not reactive with the normal human cells, normal broken cells or their membrane fragments, are then cloned twice by the limiting dilution method.

From the resultant hybridoma cells, those having a stable and high antibody titre with respect to tumour-specific antigens are selected for the preparation of monoclonal antibodies according to our invention.

(4)  Preparation of tumour-specific monoclonal antibodies:

A BALB/c mouse is abdominally administered with 2,6,10,14-tetramethylpentadecane (pristane) in an amount of 0.5 ml and bred for 2 weeks to obtain a pristane-treated mouse of age 8-10 weeks.  $2-4 \times 10^6$ cells of the selected hybridoma cells obtained by step (3) are abdominally injected into the mouse and 10 to 21 days later the ascites fluid from the tumour induced by the administered hybridoma cells is collected. The fluid is centrifuged to remove solid impurities (3000 r.p.m./5 min.) and the resultant supernatant is subjected twice to salting-out using respectively 50% and 40% ammonium sulfate, followed by dialysis against a PBS solution (pH=7.2) for 1-2 days.  The residual fraction is chromatographed on, for example, DEAE Sepharose, Protein A Sepharose (Pharmacia Fine Chemicals AB., Sweden) or the like.  The IgG fractions are collected and combined to obtain purified monoclonal antibodies.

Where the monoclonal antibodies are of IgM class, after removal of solid impurities from the ascites fluid, the remaining solution is dialyzed, for example, against dionized water, 0.01M phosphate-buffered solution (pH=6.0) or the like for 2-4 hours. The resultant precipitates are collected by centrifugation and dissolved in 3-5% sodium chloride solution, which is dialyzed against 0.85% sodium chloride solution for 1-2 days.  The residual fraction is chromatographed, for example, on DEAE Sepharose, Protein A Sepharose (Pharmacia Fine Chemicals AB., Sweden) or the like and the resultant IgM fractions are collected and combined to obtain purified monoclonal antibodies.

The isotypes of the antibodies may be investigated by Ouchterlony's method (the double diffusion method) with reference to "Meneki-gaku Jikken-ho Nvumon, Seibutsu Kagaku Jikken-ho 15", published by Gakkai Shuppan Centre, Tokyo, p. 74 (1981).

The assay of Ig protein may be effected by Folin's method with reference to the optical density at 280 nm [1.4 ($OD_{280}$) = immunoglobulin approx. 1 mg/ml].

The specificities of the resultant monoclonal antibodies may be investigated, for example, with reference to their reactivities with the specific antigens present in normal or tumour cells, broken cells, tissues or membrane fragments of such cells or tissues, originating from various organs obtained from different humans; their reactivities with the specific antigens present in various cell lines, either normal or tumour cells, or present in foetal human cells or the membrane fragments of such cell lines; their reactivities with the known carcinoembryonic antigen (for example CEA); their reactivities with the sera obtained from normal humans and from human patients having lung tumours; and so on. Reactivities may be assayed, for example, by the enzyme immunosorbent assay method, fluorescence antibody method, or by an immuno-histological method (PAP) or the like. In all cases, antibodies which are linked specifically to antigens from human lung squamous cell carcinoma and/or from human lung adenocarcinoma are selected.

The tumour-specific monoclonal antibodies of the present invention are expected to be useful, for example, for diagnosis of human lung cancer by examination of serum or by histological examination: Antibodies labelled with e.g. radioactive or fluorescent agents may be useful for tumour imaging and the like. Moreover, the monoclonal antibodies of the present invention are of interest in the treatment of human lung cancer by administration of the antibodies per se or by administration as a so-called immunotoxin derivative, i.e. an agent in which the antibodies are combined with an anti-tumour or cytotoxic agent.

The tumour-specific antibodies may also be of use to make an affinity column, useful for the purification of tumour-specific antigens, the analysis of such antigens, and development of anti-human lung cancer vaccines.

It will be understood that for some purposes the antigen-binding fragments of monoclonal antibodies according to our invention may be of comparable utility, and references to monoclonal antibodies herein should be understood as extending to antigen-binding fragments thereof where applicable.

The following non-limiting examples illustrate the present invention.

Example 1

(1)    Preparation of antibody-forming cells

BALB/c mice (age 8 weeks) were abdominally administered with membrane fragments of human lung squamous cell carcinoma (100 mcg protein per aminal) in association with aluminium hydroxide gel (2 mg per animal) and pertussis adjuvant ($1 \times 10^9$ cells per animal). Follow-up immunizations were effected using a same antigen (100 mcg protein per animal) 3-5 times at intervals of 1-2 weeks. Antiserum was collected from each animal to investigate the reactivity of the antiserum with a specific antigen present in the cells, tissues or membrane fragments of such cells or tissues, originating from human lung squamous cell carcinoma. The animals which exhibited strong reactivity were deemed to be efficiently immunised and their spleen cells were used as antibody-forming cells.

(2)    Preparation of myeloma cells

A myeloma cell line originating from the 8-azaguanine resistant mouse (P3-U1) were cultured in normal medium to obtain more than $2 \times 10^7$ cells. The resultant cells were used for fusion.

(3)    Preparation of hybridoma cells

The spleen cells and myeloma cells obtained by the steps (1) and (2) respectively were mixed in a ratio of 5:1 and fused together by the method as hereinbefore described. A HAT medium was used for culturing the fused cells at 37°C for 14 days under a 5% $CO_2$ atmosphere. The growing hybridoma colonies were selected and further cultured in HT medium. The antibody titres of the cells were determined with respect to human lung squamous cell carcinoma-specific antigen to select the wells with active cells, which were then transferred to normal medium followed by cloning twice.

By using various assay methods, two monoclones designated as SLC-1 and SLC-2 were selected. SLC-1 was capable of producing monoclonal antibodies which are unreactive with specific antigens in normal human cells, normal tissues and other human cancers, but specifically reactive with a specific antigen in human lung squamous cell carcinoma; SLC-2 was capable of producing monoclonal antibodies which are reactive with tumour-specific antigens from both human lung squamous cell carcinoma and human lung adenocarcinoma.

(4)    Purification of monoclonal antibodies

The hybridoma cells obtained in step (3) were abdominally injected into pristane-treated BALB/c female mice (age 8 weeks) at a dose of $4 \times 10^6$ cells per animal. 10-21 days later, ascites fluid from the induced tumour was collected from the mice (5-10 ml per animal) and the combined fluid was centrifuged (3000 r.p.m./5 min.) to remove solid impurites. The remaining solution was dialyzed against deionized water or a 0.01M PBS solution (pH=6.5) for 2 hours. The resultant precipitated fraction was centrifuged (10000 r.p.m./15 min.) to remove supernatant. The material was dissolved in a 3% sodium chloride solution and dialyzed against 0.85% sodium chloride solution for 2 days to yield a partially purified monoclonal antibody solution (IgM class).

Table 1 shows the chracteristics of the resultant monoclonal antibodies SLC-7 and SLC-8 respectively specific to human lung squamous cell carcinoma and to both human lung squamous cell carcinoma and adenocarcinoma.

Example 2

A similar procedure to that described in Example 1 was carried out except that membrane fragments of human lung adenocarcinoma were used in the immunisation. The hybridoma finally selected produces a monoclonal antibody designated AL-5, which is a human lung adenocarcinoma-specific monoclonal antibody. The characteristics of ALC-5 are shown in Table 2.

### TABLE 1

| | Antibody*** | |
| --- | --- | --- |
| | SLC-7 | SLC-8 |
| Analysis of binding (ELISA) | | |
| Tissue | | |
| Lung squamous cell carcinoma | 6/8 | 5/8 |
| Lung adenocarcinoma | 0/8 | 4/8 |
| Normal lung | 0/6 | 1/6 |
| Normal tissue of* | 0/9 | 0/9 |
| Carcinoembryonic antigen (CEA) | - | - |
| Cell lines | | |
| Lung squamous cell carcinoma | 1/2 | 1/2 |
| Lung adenocarcinoma | 0/5 | 3/5 |
| Foetal lung | 0/3 | 0/3 |
| Cancers other than lung cancer** | 0/8 | 0/8 |
| Normal lung | 0/2 | 0/2 |
| Tissue dyeing | | |
| Lung squamous cell carcinoma | 5/5 | 4/5 |
| Lung adenocarcinoma | 0/5 | 3/5 |
| Normal lung | 0/5 | 0/5 |
| Fluorescence antibody method | | |
| Lung squamous cell carcinoma | 1/2 | 1/2 |
| Lung adenocarcinoma | 0/5 | 3/5 |
| Normal lung | 0/1 | 0/1 |
| Antibody class | IgM | IgM |

* Normal stomach, liver, kidney, spleen, heart and large intestine.

** Cancers of stomach and large intestine, melanoma, uterine cancer, trophoblastic tumour, ganglioma, leukaemia and myeloma.

*** positive case/test cases

0155172

TABLE 2

| | Antibody ***<br>ALC-5 |
|---|---|
| Analysis of binding (ELISA) | |
| **Tissue** | |
| Lung squamous cell carcinoma | 0/8 |
| Lung adenocarcinoma | 5/8 |
| Normal lung | 0/6 |
| Normal tissue of* | 0/9 |
| Carcinoembryonic antigen (CEA) | – |
| | |
| **Cell lines** | |
| Lung squamous cell carcinoma | 0/2 |
| Lung adenocarcinoma | 4/5 |
| Foetal lung | 0/3 |
| Cancers other than lung cancer** | 0/8 |
| Normal lung | 0/2 |
| **Tissue dyeing** | |
| Lung squamous cell carcinoma | 0/5 |
| Lung adenocarcinoma | 4/5 |
| Normal lung | 0/5 |
| **Fluorescence antibody method** | |
| Lung squamous cell carcinoma | 0/2 |
| Lung adenocarcinoma | 4/5 |
| Normal lung | 0/1 |
| **Antibody class** | IgM |

---

\*  Normal stomach, liver, kidney, spleen, heart and large intestine.

\*\*  Cancers of stomach and large intestine, melanoma, uterine cancer, trophoblastic tumour, ganglioma, leukaemia and myeloma.

\*\*\*  positive case/test cases

---

- 15 -          0155172

CLAIMS:

1.    A human lung tumour-specific monoclonal antibody which binds to an antigen specific to human lung tumour but not to an antigen of normal human tissue of the lung or to an antigen of other organs.

2.    An antibody according to claim 1 which binds to an antigen of squamous cell carcinoma of the human lung but not to an antigen of adenocarcinoma of the human lung.

3.    An antibody according to claim 1 which binds to an antigen of adenocarcinoma of the human lung but not to an antigen of squamous cell carcinoma of the human lung.

4.    An antigen-binding fragment of an antibody according to any of claims 1 to 3.

5.    An antibody according to any of claims 1 to 3 or the antigen-binding fragment according to claim 4 linked to an antitumour agent.

6.    An antibody according to any of claims 1 to 3 or the antigen-binding fragment according to claim 4 linked to a tracer or imaging agent.

7.    A hybridoma which secretes an antibody according to any of claims 1 to 3.

8.    A process for producing the hybridoma of claim 7 which comprises immunizing an animal with human lung cancer antigens originating from human lung squamous cell carcinoma and/or human lung adenocarcinoma; fusing antibody-producing cells from said immunized mammal with myeloma cells to produce hybridoma cells; and selecting from the resultant hybridoma cells those which produce monoclonal antibodies having the desired binding specificity.

9.    A process according to claim 8 wherein said myeloma cells are a myeloma cell line of mouse origin.

10.   A method for the production of a monoclonal antibody according to any of claims 1 to 3 which comprises culturing the hybridoma of claim 7, in vitro or in vivo, and recovering said antibody from the culture.